# EUROPEAN PATENT APPLICATION

(11) **EP 1 813 285 A1**
(43) Date of publication of application: **01.08.2007**
(21) Application number: 05806315.7
(22) Date of filing: 14.11.2005
(51) Int. Cl.: A61K 45/06, A61K 45/00, A61K 31/137, A61K 31/165, A61K 31/4168, A61P 29/00

(54) **PREVENTIVE OR THERAPEUTIC AGENT FOR NEUROPATHIC PAIN**

(30) Priority: 19.11.2004 JP 2004335510
(71) Applicant: Kissei Pharmaceutical Co., Ltd., Matsumoto-shi Nagano 399-8710 (JP)
(72) Inventor: KOBAYASHI, Mamoru, c/o Kissei Pharma. Co. Ltd., Azumino-shi, Nagano 399 (JP); KIGUCHI, Sumiyoshi, c/o Kissei Pharma. Co. Ltd., Azumino-shi, Nagano 399 (JP)
(74) Representative: Hayes, Adrian Chetwynd
(86) International application number: PCT/JP2005/020830
(87) International publication number: WO 2006/054513

(57) **Abstract**

The present invention provides medicinal agents that are useful for the prevention or treatment of neuropathic pain which comprises as an active ingredient a β2 adrenoceptor stimulant. In addition, the present invention provides formulations for the prevention or treatment of neuropathic pain such as painful diabetic neuropathy, postherpetic neuralgia, trigeminal neuralgia, or postoperative or traumatic chronic pain, that are **characterized by** the use in combination of an α₂-adrenoceptor stimulant and a β₂-adrenoceptor stimulant, or by containing a compound that has both α₂-adrenoceptor stimulation and β₂-adrenoceptor stimulation activities as an active ingredient or the like.

## Description

### Field of the invention

The present invention relates to formulations that are useful for the prevention or treatment of neuropathic pain.

More specifically, the present invention relates to a formulation for the prevention or treatment of neuropathic pain that comprises as an active ingredient a β₂-adrenoceptor (hereinafter referred to as β₂ AR) stimulant, a combination formulation for the prevention or treatment of neuropathic pain that are characterized by comprising an α₂-adrenoceptor (hereinafter referred to as α₂ AR) stimulant and a β₂-AR stimulant and the like.

### Background Art

Neuropathic pain is defined as pain caused or induced when the nervous system is injured temporarily or is in dysfunction. The pain is an intractable algetic disease, as it is resistant to antiphlogistic analgesics and anesthetic analgesics. The typical diseases include cancerous pain, postherpetic neuralgia, trigeminal neuralgia, phantom limb pain, causalgia and painful diabetic neuropathy (or diabetic painful neuropathy) and the like. Among neuropathic pain diseases, particularly prevalent is painful diabetic neuropathy. It is conjectured that the number of such patients will increase further as that of diabetic patients increases along with changes in life style and aging of the population. Patients with the above-mentioned diseases have pain and sensory abnormality characterized by hyperalgesia and allodynia. It has been reported that because said symptoms persist, patients often suffer from insomnia, loss of appetite or reactive depression, markedly affecting adversely the QOL of patients (for example, see Non-patent reference 1).

The etiology of neuropathic pain remains mostly unknown. It is conjectured that the disease is induced partially by peripheral and central neuropathy at various levels, and pain is manifested as a metabolic abnormality, either at peripherally or centrally, blood flow disorder and degeneration of nerve fiber, and changes in synaptic responsiveness lie complexly one upon another.

Neuropathic pain is treated with pharmacotherapy and nerve block therapy. Drugs used in pharmacotherapy include anticonvulsants, psychotropic vitamins, non-steroidal anti-inflammatory drugs, aldose reductase inhibitors, hypoglycemic drugs and lidocaine-like antiarrhythmic drugs. Nerve block therapy includes stellate nerve block, continuous epidural block, nerve root block and the like. However, because hypersusceptibility to neuropathic pain is caused by the breakdown of balance between conduction system and suppression system of pain, these treatments are often insufficiently effective. Quick development of new drugs is hoped.

It is known that α₂ AR stimulants lower blood pressure and peripheral vascular resistance and are useful for the treatment of hypertension, cancerous pain by epidural administration (that is, epidural block), postoperative pain and so on (for example, see Non-patent reference 2). It was reported that α₂ AR stimulants demonstrated analgesic action in rats with peripheral nerve disorders (Non-patent reference 3). However, α₂ AR stimulants are not used practically as the systemic therapeutic agent for neuropathic pain partly because of problems of central adverse effects, particularly, sedation, sleepiness, dizziness, thirstiness and so on.

It is known that β₂ AR stimulants suppress smooth muscle contraction and so on and are useful as bronchodilator, the therapeutic agent of imminent abortion and premature labor, pain relief and lithagogue for uretero-lithiasis and so on (for example, see Patent reference 1 and Non-patent reference 4). It was also reported that a β₂ AR stimulant improved nerve blood flow in rat diabetic neuropathy models (Non-patent reference 5). However, there is no report that β₂ AR stimulants are effective analgesics for neuropathic pain. Furthermore, as it has been suggested that β₂ AR stimulants at high doses may increase blood sugar levels in diabetic patients; no studies have been conducted on β₂ AR stimulants as painkiller for diabetic neuropathy.

As mentioned above, it is not known at all that β₂ AR stimulants alleviate neuropathic pain, or a combination of an α₂ AR stimulant and a β₂ AR stimulant is more effective for neuropathic pain than the sole use of either agent.

[Patent reference 1] International publication No. 97 - 30023 pamphlet
[Non-patent reference 1] Katsuyuki Moriwaki et al.: Pain Clinic, Vol. 21, May 2000, Supplement pp.S101-S107
[Non-patent reference 2] Shuji Dohi.: The Japanese Journal of Clinical Medicine, 2001, Vol. 59, pp.1800-1805
[Non-patent reference 3] Frederic Duflo et al.: Anesthesiology, 2002, Vol. 97, pp.636-641
[Non-patent reference 4] Chikako Tanaka et al. (ed) New Pharmacology, pp.227-236, 2002 (Nankodo)
[Non-patent reference 5] Mary A. Cotter et al.: European Journal of Pharmacology, 1998, Vol. 343, pp.217-223

### Disclosure of the invention

### Problem that the invention aims to solve

The purpose of the present invention is to provide therapeutic formulations for neuropathic pain.

### Means to solve the problem

As the result of strenuous research on the above-mentioned problem, the inventors found, to our surprise, that, by administering a β₂ AR stimulant, the combination of an α₂ AR stimulant and a β₂ AR stimulant or a compound that has both α₂ AR stimulation and β₂ AR stimulation activities, neuropathic pain was alleviated in diabetic rats induced by streptozotocin (hereinafter referred to as STZ) and Seltzer model rats, and thereby forming the basis of the present invention.

That is, the present invention relates to:
[1] a formulation for the prevention or treatment of neuropathic pain, which comprises a β₂-adrenoceptor stimulant as an active ingredient;
[2] a formulation for the prevention or treatment of neuropathic pain as described in the above [1], which comprises the combination of an α₂-adrenoceptor stimulant and a β₂-adrenoceptor stimulant;
[3] a formulation as described in the above [2], which comprises as an active ingredient a compound that has both α₂-adrenoceptor stimulation and β₂-adrenoceptor stimulation activities;
[4] a formulation as described in the above in any of the above [1] to [3], wherein neuropathic pain is painful diabetic neuropathy, postherpetic neuralgia, trigeminal neuralgia, phantom limb pain, causalgia, cancerous pain, or postoperative or traumatic chronic pain;
[5] a formulation as described in any of the above [1] to [4], which can be used in combination with one or more of drugs selected from a group consisting of a psychotropic vitamins, a non-steroidal anti-inflammatory drug, an aldose reductase inhibitor, a lidocaine-like anti-arrhythmic drug, an antidepressant and an anticonvulsant;
[6] a method of the prevention or treatment of neuropathic pain, which comprises repeated administration of effective doses of a β₂-adrenoceptor stimulant for 2 weeks or longer;
[7] a method of the prevention or treatment of neuropathic pain, which comprises administration of effective doses of the combination of an α₂-adrenoceptor stimulant and a β₂-adrenoceptor stimulant;
[8] a use of a β₂-adrenoceptor stimulant for the manufacture of a formulation for the prevention or treatment of neuropathic pain;
[9] a use of an α₂-adrenoceptor stimulant and a β₂-adrenoceptor stimulant for the manufacture of a formulation the prevention or treatment of neuropathic pain; and the like.

### Effect of the invention

Combination formulations of the present invention which contain an α₂ AR stimulant and a β₂ AR stimulant demonstrated extremely effective analgesic action in STZ-induced diabetic rats and Seltzer model rats, and are therefore useful for the prevention or treatment of neuropathic pain.

### Brief description of the drawing

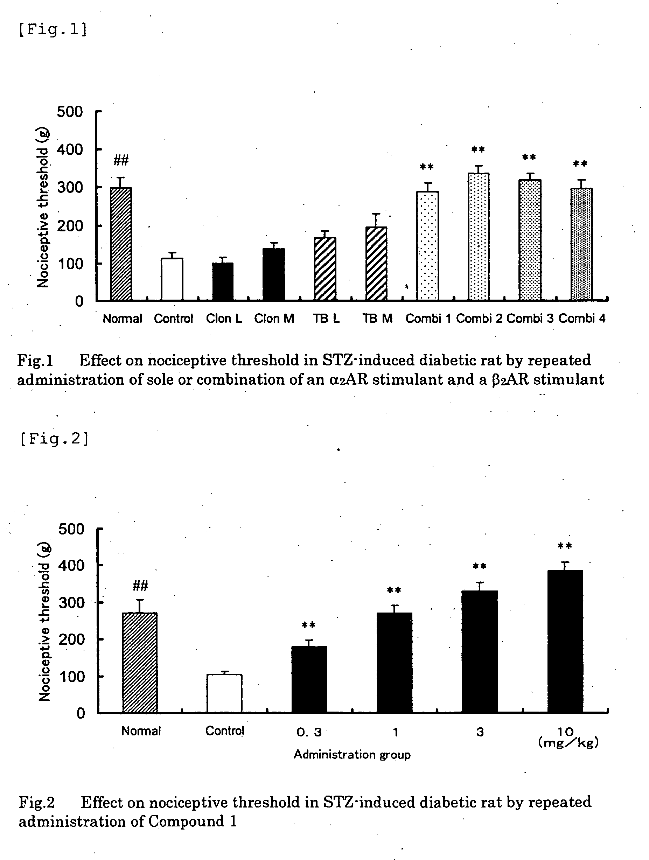

[Figure 1] Analgesic effects of sole and combination administrations (in repeated administration) of an α₂ AR stimulant and a β₂ AR stimulant in STZ-induced diabetic rats are shown. The axis of abscissas in the figure denotes administration groups, wherein Normal indicates a normal group, Control indicates a control group, Clon L indicates a clonidine L group (0.1 mg/kg), Clon M indicates a clonidine M group (0.3 mg/kg), TB L indicates a terbutaline L group (1 mg/kg), TB M indicates a terbutaline M group (3 mg/kg), Combi1 indicates a combination L-L group (combination of clonidine 0.1 mg/kg and terbutaline 1 mg/kg), Combi2 indicates a combination L-M group (combination of clonidine 0.1 mg/kg and terbutaline 3 mg/kg), Combi3 indicates a combination M-L group (combination of clonidine 0.3 mg/kg and terbutaline 1 mg/kg), and Combi4 indicates a combination M-M group (combination of clonidine 0.3 mg/kg and terbutaline 3 mg/kg). The symbol "**" means P<0.01 (significant difference from Control in Steel test), and "##" indicates P<0.01 (significant difference from Control in Aspin-Welch's t test).

[Figure 2] Analgesic effects of a compound which has both α₂ AR stimulation and β₂ AR stimulation activities (in repeated administration) in STZ-induced diabetic rats are shown. The axis of abscissas in the figure denotes administration groups, wherein Normal and Control have the same meanings as described in the above Figure 1. Numerals denote doses of compound 1 (mg/kg). The symbols "**" and "##" denote the same meanings as described in the above Figure 1.

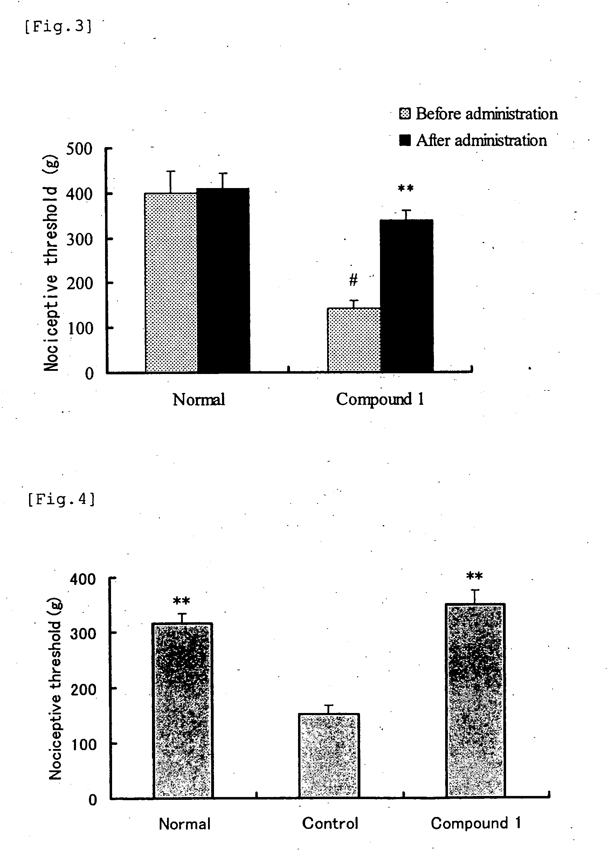

[Figure 3] Analgesic effects of a compound which has both a₂ AR stimulation and β₂ AR stimulation activities (in single administration) in Seltzer model are shown. The axis of abscissas in the figure denotes administration groups wherein Normal is the normal group. The symbol "**" indicates P<0.01 (significant difference from the pre-administration value (two corresponding groups were tested). The symbol "#" indicates P<0.05 (significant difference in pre-administration values between normal group and nerve ligation group in Aspin-Welch's t test).

[Figure 4] Analgesic effects of a compound which has both α₂ AR stimulation and β₂ AR stimulation (in repeated administration) in Seltzer model are shown. The axis of abscissas in the figure denotes administration groups, wherein Normal and Control have the same meanings as described in the above Figure 1. The symbol "**" indicates P<0.01 (significant difference from the control group in Aspin-Welch's t test).

### Best mode to operate the invention

As α₂ AR stimulants; any known α₂ AR stimulant may be used. Such agents include, for example, clonidine, moxonidine, rilmenidine, medetomidine, dexmedetomidine, guanfacine, guanabenz, α-methylnoradrenaline, methyldopa, UK14304, B-HT920 and B-HT933.

A dosage of an α₂ AR stimulant may be determined as needed according to individual α₂ AR stimulant, patients' body weight, age, sex and seriousness of diseases. For example, the range of dosages of the drugs in oral administration to adults can be approximately 0.01 to 0.45 mg/day of clonidine hydrochloride, 0.01 to 0.45 mg/day of moxonidine, 0.05 to 2.0 mg/day of rilmenidine, 0.01 to 0.45 mg/day of medetomidine, 0.01 to 0.45 mg/day of dexmedetomidine, 0.01 to 1.5 mg/day of guanfacine hydrochloride, 0.1 to 20.0 mg/day of guanabenz, 0.01 to 10.0 mg/day of α-methylnoradrenaline, 10.0 to 2,500 mg/day of methyldopa, 0.01 to 0.45 mg/day of UK14304, 0.01 to 0.45 mg/day of B-HT920 and 0.1 to 4.5 mg/day of B-HT933.

As β₂ AR stimulants, any known β₂ AR stimulant may be used. Such drugs include, for example, procaterol, ritodrine, terbutaline, salbutamol, clenbuterol, tulobuterol, mabuterol, salmeterol, formoterol, isoprenaline, trimetoquinol, hexoprenaline, methoxyphenamine, orciprenaline and fenoterol.

A dosage of a β₂ AR stimulant may be determined as needed according to individual β₂ AR stimulant, patients' body weight, age, sex and seriousness of diseases. For example, the range of dosages of the drugs in oral administration to adults can be approximately 0.001 to 0.2 mg/day of procaterol hydrochloride, 0.01 to 150 mg/day of ritodrine hydrochloride, 0.01 to 15 mg/day of terbutaline sulfate, 0.01 to 15 mg/day of salbutamol sulfate, 0.001 to 0.1 mg/day of clenbuterol hydrochloride, 0.1 to 10 mg/day of tulobuterol hydrochloride, 0.01 to 0.1 mg/day of mabuterol hydrochloride, 0.01 to 0.1 mg/day of salmeterol xinafoate, 0.01 to 0.2 mg/day of formoterol fumarate, 0.1 to 15 mg/day of isoprenaline hydrochloride, 0.1 to 20 mg./day of trimetoquinol hydrochloride, 0.001 to 0.02 mg/day of hexoprenaline, 10 to 300 mg/day of methoxyphenamine hydrochloride, 0.5 to 100 mg/day of orciprenaline sulfate and 0.1 to 10 mg/day of fenoterol hydrobromide. The dosages of β₂ AR stimulants used may be also reduced when used in combination with α₂ AR stimulants.

A combination formulation of the present invention that contains an α₂ AR stimulant and a β₂ AR stimulant includes a single formulation containing an α₂ AR stimulant and a β₂ AR stimulant, a single formulation containing a compound that has both α₂ AR stimulation and β₂ AR stimulation activities, a formulation in a package that contains the combination of a formulation containing an α₂ AR stimulant and a formulation containing a β₂ AR stimulant, and a combination of a formulation containing an α₂ AR stimulant and a formulation containing a β₂ AR stimulant that are co-administered simultaneously or at intervals in the same administration form or different administration forms.

As the compounds that have both α₂ AR stimulation and β₂ AR stimulation activities of the present invention, a compound which has the binding ability to α₂ AR no greater than 10⁻⁴ mol/L as the IC₅₀ value and the binding ability to β₂ AR no greater than 10⁻⁵ mol/L as the IC₅₀ value is preferable. An example of such a compound is (-)-bis(2-[(2S)-2-[{(2R)-2-hydroxy-2-(4-hydroxy-3-hydroxymethylphenyl)ethyl}amino]-1,2,3,4-tetrahydronaphthalen-7-yloxy]-N,N-dimethylacetamide} sulfate dihydrate (hereinafter referred to as Compound 1). The Compound 1 can be manufactured easily in accordance with methods described in literatures (for example, see Patent reference 1).

Combination formulations of the present invention markedly increase the nociceptive threshold in models such as STZ-induced diabetic rats, which is the representative model for evaluation of drug efficacy in neuropathic pain, and therefore, are useful for the prevention or treatment of neuropathic pain. Neuropathic pain includes, for example, painful diabetic neuropathy, postherpetic neuralgia, trigeminal neuralgia, phantom limb pain, causalgia, cancerous pain and postoperative or traumatic chronic pain.

Of combination formulations of the present invention, a single formulation containing an α₂ AR stimulant and a β₂ AR stimulant can be manufactured by admixing or by diluting and dissolving an α₂ AR stimulant and a β₂ AR stimulant with formulation carriers including necessary excipients, disintegrators, binders, lubricants, diluents, buffers, isotonic agents, antiseptics, humectants, emulsifiers, dispersing agents, stabilizers and solubilizers or the like in various dosage forms in the usual way. When an α₂ AR stimulant and a β₂ AR stimulant are administered as separate formulations, single formulations of each agent that are available may be used.

Examples of administration forms of the pharmaceutical composition of the present invention are forms administered orally such as powders, granules, fine granules, dry syrup, tablets, capsules or the like, forms administered non-orally such as injections, poultices, suppositories or the like. Forms administered orally is preferable.

A pharmaceutical composition of the present invention may be used occasionally in combination with other drugs that have effects alleviating symptoms of neuropathic pain. Examples of other drugs that have effects alleviating symptoms of neuropathic pain include psychotropic vitamins such as vitamin B₁₂ and so on; non-steroidal anti-inflammatory drugs such as indomethacin, diclofenac and so on; aldose reductase inhibitors such as epalrestat and so on; lidocaine-like antiarrhythmic drugs such as mexiletine, lidocaine and so on; antidepressants such as imipramine, amitriptine, mianserin and so on; and anticonvulsants such as carbamazepine, phenytoin and so on.

### Example

The present invention is further illustrated in more detail by way of the following Examples. However, the present invention is not limited thereto.

### [Test Example 1] Measurement of the nociceptive threshold in STZ-induced diabetic rats (single administration)

STZ (50 mg/kg) was administered intravenously to male SD stain rats to induce diabetes mellitus. Test articles were administered 14 days later (10 rats in each group). Test articles used were clonidine as the a₂ AR stimulant, terbutaline as the β₂ AR stimulant and the above-mentioned Compound 1. Normal and Control groups received the medium (0.5% methylcellulose). Before and one hour after administration of test articles, the nociceptive threshold against pressure stimulation given to the right hind-paw of rats was measured by Randall-Selitto method, and compared with that in Control group.

### [Test Example 2] Measurement of the nociceptive threshold in STZ-induced diabetic rats (repeated administration)

STZ (50 mg/kg) was administered intravenously to male SD stain rats to induce diabetes mellitus. Beginning on the following day of STZ administration, each test article was administered orally once a day, repeatedly, 14 times in total (9 to 10 rats in each group). Normal and Control groups received the medium (0.5% methylcellulose). Before the final administration (before administration of test articles) and after the final administration (one hour after the administration), the nociceptive threshold against pressure stimulation given to the right hind-paw of rats was measured by Randall-Selitto method, and compared with that in Control group.

### [Test Example 3] Measurement of the nociceptive threshold in Seltzer model rats (single administration)

Ten, 9-week old male SD rats were anesthetized with pentobarbital (Nembutal injection®), and the right sciatic nerve was exposed. In Nerve ligation group (7 rats), half of the dorsal portion of the nerve was ligated using 5-0 nylon thread. In Normal group (3 rats), the sciatic nerve was only exposed. Three weeks after the model was created, the control group received the medium (0.5% methylcellulose) and Nerve ligation group was given orally test articles. Before the administration (before administration of test articles) and after administration (one hour after the administration of test articles), the nociceptive threshold against pressure stimulation given to the right hind-paw of rats was measured by Randall-Selitto method. Results before and after administration were compared.

### [Test Example 4] Measurement of the nociceptive threshold in Seltzer model rats (repeated administration)

Thirty, 7-week old male SD rats were anesthetized with pentobarbital (Nembutal injection®), and the right sciatic nerve was exposed. In the nerve ligation group (20 rats), half of the dorsal portion of the nerve was ligated using 6-0 silk thread. In the normal group (10 rats), the sciatic nerve was only exposed. Beginning the following day of the model creation, test articles were administered orally once a day, repeatedly, 14 times in total. Normal and Control groups received the medium (0.5% methyl-cellulose) and Nerve ligation group was given orally test articles. One hour after the final administration, the nociceptive threshold against pressure stimulation given to the right hind-paw of rats was measured by Randall-Selitto method, and compared with Control group.

### [Test Example 5] Receptor binding tests of α₂ AR and β₂ AR

Receptor binding tests were conducted in ³H-p-aminoclonidine and ³H-dihydroalprenolol (3 rats in each) using specimens of the membrane of rat cerebral cortex (α₂ AR) and the fascia of gravid uterus (β₂ AR) as the sources of receptors. The binding ability of Compound 1 to α₂ AR and β₂ AR were obtained by calculating binding inhibition rates (IC₅₀ values) of Compound 1 against the binding abilities of the above-mentioned tracers with both receptors. As a result, the binding affinity (IC₅₀ value) of Compound 1 to α₂ AR and β₂ AR was, in concentration, 5.8 x10⁻⁷ mol/L and 1.4x10⁻⁸ mol/L, respectively.

### [Example 1] Analgesic effects of α₂ AR stimulant and β₂ AR stimulant (single administration) in STZ-induced diabetic rats

In accordance with the method of Test Example 1, studied were analgesic effects of clonidine (L group: 0.1 mg/kg; H group: 1.0 mg/kg, administered by subcutaneous injection) and terbutaline (L group: 1 mg/kg; H group: 10 mg/kg, oral administration). Table 1 shows the nociceptive threshold (mean±SE) before and after administration of each group. In the table, the symbol "##" indicates P<0.01: significant difference from Control group (in Aspin-Welch's t test), and the symbol "**" indicates P<0.01: significant difference from Control group (in Steel test).

As a result, the nociceptive thresholds before administration were significantly lower in Control and Test article groups than in Normal group. There was no difference between Control and Test article groups. The nociceptive threshold after administration increased in clonidine H group, demonstrating an apparent analgesic effect in this dose compared with Control group.

[Table 1]

**Table 1. Nociceptive threshold in STZ-induced diabetic rats (single administration)**

| Administration group | Dose (mg/ kg) | Nociceptive threshold before administration (g) | Nociceptive threshold after administration (g) |
|---|---|---|---|
| Normal group | - | 302.6±15.7^{##} | 286.4±21.5^{##} |
| Control group | - | 119.2±11.7 | 121.4±11.8 |
| Clonidine L group | 0.1 | 122.8±7.5 | 155.8±16.3 |
| Clonidine H group | 1.0 | 115.4±8.9 | 325.8±29.8** |
| Terbutaline L group | 1.0 | 134.2±6.3 | 152.8±12.7 |
| Terbutaline H group | 10.0 | 122.6±14.5 | 146.8±20.7 |

### [Example 2] Analgesic effects of α₂ AR stimulant and β₂ AR stimulant (repeated administration) in STZ-induced diabetic rats

In accordance with the method of Test Example 2, studied were analgesic effects of repeatedly administered clonidine (L group: 0.1 mg/kg; M group: 0.3 mg/kg; H. group: 1.0,mg/kg, administered by subcutaneous injection) and terbutaline (L group: 1 mg/kg; M group: 3 mg/kg; H group: 10 mg/kg; oral administration). Table 2 shows the nociceptive threshold (mean±SE) after administration in each group. In the table, the symbol "##" indicates P<0.01: significant difference from Control group (in Aspin-Welch's t test), and the symbol "*" indicates P<0.05 and "**" indicates P<0.01: significant differences from Control group (in Steel test).

As a result, following repeated administration of clonidine or terbutaline for 14 days, the nociceptive threshold increased significantly only in clonidine H group and terbutaline H group.

[Table 2]

**Table 2. Nociceptive threshold in STZ-induced diabetic rats (repeated administration)**

| Administration group | Dose (mg/kg) | Nociceptive threshold before administration (g) | Nociceptive threshold after administration (g) |
|---|---|---|---|
| Normal group | - | 297.6±25.2^{##} | 306.6±26.9^{##} |
| Control group | - | 109.6±8.7 | 119.8±13.7 |
| Clonidine L group | 0.1 | 129.2±15.9 | 180.6±24.4 |
| Clonidine M group | 0.3 | 104.2±13.8 | 115.2±18.2 |
| Clonidine H group | 1.0 | 97.2±14.5 | 254.4±33.3* |
| Terbutaline L group | 1.0 | 113.8±11.9 | 115.2±12.6 |
| Terbutaline M group | 3.0 | 155.4±32.0 | 167.8±32.3 |
| Terbutaline H group | 10.0 | 228.2±11.1** | 229.0±14.8** |

### [Example 3] Analgesic effects of α₂ AR stimulant and β₂ AR stimulant used solely and in combination (repeated administration) in STZ-induced diabetic rats

In accordance with the method of Test Example 2, studied were analgesic effects of repeatedly administered clonidine (L group: 0.1 mg/kg; M group: 0.3 mg/kg, administered by subcutaneous injection), terbutaline (L group:1 mg/kg; M group: 3 mg/kg, oral administration), and combination (L-L group: co-administration of clonidine 0.1 mg/kg and terbutaline 1 mg/kg; L-M group: co-administration of clonidine 0.1 mg/kg and terbutaline 3 mg/kg, group; M-L group: co-administration of clonidine 0.3 mg/kg and terbutaline 1 mg/kg; and M-M group: co-administration of clonidine 0.3 mg/kg and terbutaline 3 mg/kg). Figure 1 shows the nociceptive threshold (mean±SE) after administration in each group.

As a result, after a 14-day repeated, sole administration of clonidine or terbutaline, in neither L groups or M groups of either test articles, the nociceptive threshold increased. While, in all combination groups comprising in combination each dose that solely had no action clearly increased nociceptive thresholds. The effects were synergistic, exceeding sum of effects in nociceptive threshold in sole administration of each agent. In all combination groups, nociceptive thresholds improved nearly to that in Normal group.

### [Example 4] Analgesic effects of a compound that has both α₂ AR stimulation and β₂ AR stimulation activities (single administration) in.STZ-induced diabetic rats

In accordance with the method of Test Example 1, studied was anti-nociceptive action of Compound 1 (0.3, 1, 3 and 10 mg/kg, oral administration). Table 3 shows nociceptive thresholds (mean±SE) after administration in each group. In the table, the symbol "#" indicates P<0.05: significant difference from Control group (in Aspin-Welch's t test), and the symbol "*" indicates P<0.05: significant difference from Control group (in Steel test).

As a result, in each group receiving Compound 1, the nociceptive thresholds after administration increased dose-dependently. Groups receiving 3 mg/kg and 10 mg/kg of Compound 1 demonstrated apparent analgesic effects.

[Table 3]

**Table 3. Nociceptive threshold in STZ-induced diabetic rats (single administration)**

| Administration group | Dose (mg/ kg) | Nociceptive threshold before administration (g) | Nociceptive threshold after administration (g) |
|---|---|---|---|
| Normal group | - | 298.2±35.7^{#} | 283.2±28.1^{#} |
| Control group | - | 86.8±7.7 | 91.8±8.7 |
| Compound 1 | 0.3 | 71.8±5.3 | 117.6±10.7 |
| Compound 1 | 1.0 | 95.0±5.1 | 131.0±12.3 |
| Compound 1 | 3.0 | 92.0±13.3 | 174.6±15.4* |
| Compound 1 | 10.0 | 89.0±10.2 | 253.4±28.3* |

### [Example 5] Analgesic effects of a compound that has both α₂ AR stimulation and β₂ AR stimulation activities (repeated administration) in.STZ-induced diabetic rats

In accordance with the method of Test Example 2, studied were analgesic effects of repeatedly administered Compound 1 (0.3, 1, 3 and 10 mg/kg, oral administration). Figure 2 shows nociceptive thresholds (mean±SE) after Administration in each group.

As a result, after a 14-day repeated oral administration of Compound 1, the nociceptive thresholds after administration clearly increased dose-dependently beginning at dosage of 0.3 mg/kg. Repeated administration of Compound 1 demonstrated stronger effect increasing the nociceptive threshold than single administration, and improved the nociceptive threshold to the same level as in Normal group. The results confirmed that a compound that has both α₂ AR stimulation and β₂ AR stimulation activities also manifests apparent analgesic effect as the co-administration of both stimulants.

### [Example 6] Analgesic effect of a compound that has both α₂ AR stimulation and β₂ AR stimulation activities (single administration) in Seltzer model

In accordance with the method of Test Example 3, studied was analgesic effect of Compound 1 (10 mg/kg, oral administration). Figure 3 shows the nociceptive thresholds (mean±SE) before and after administration in each group.

As a result, 3 weeks after model was created, the nociceptive threshold of the right hind-paw of Nerve ligation group decreased significantly compared with that of Normal group. Following administration of Compound 1, the nociceptive threshold of Nerve ligation group improved significantly in comparison with that before administration.

### [Example 7] Analgesic effect of a compound that has both α₂ AR stimulation and β₂ AR stimulation activities (repeated administration) in Seltzer model

In accordance with the method of Test Example 4, studied was analgesic effect of repeatedly administered Compound 1 (10 mg/kg, oral administrat.ion). Figure 4 shows the nociceptive thresholds (means±SE) before and after administration in each group.

As a result, following a 14-day repeated administration of Compound 1, the nociceptive threshold of the right hind-paw improved significantly in comparison with that of Control group.

As described, a β₂ AR stimulant in sole administration showed analgesic action. Particularly, when a β₂ AR stimulant was combined with an α₂ AR stimulant, the combination demonstrated synergistic analgesic action at doses each of that had no analgesic action in sole administration of either agent. Similar to the co-administration of both stimulants, a compound that had both α₂ AR stimulation and β₂ AR stimulation activities exhibited apparent analgesic action. Thus, the uses of β2 AR stimulants, particularly in combination with α₂ AR stimulants, increased markedly the nociceptive thresholds in STZ-induced diabetic rats and Seltzer model rats, and therefore, are extremely useful for the prevention or treatment of neuropathic pain.

### Industrial applicability

The pharmaceutical compositions of the present invention are extremely useful as agents.for the prevention or treatment of neuropathic pain.

## Claims

1. A formulation for the prevention or treatment of neuropathic pain, which comprises a β₂-adrenoceptor stimulant as an active ingredient.

2. A formulation for the prevention or treatment of neuropathic pain as claimed in claim 1, which comprises the combination of an α₂-adrenoceptor stimulant and a β₂-adrenoceptor stimulant.

3. A formulation as claimed in claim 2, which comprises as an active ingredient a compound that has both α₂-adrenoceptor stimulation and β₂-adrenoceptor stimulation activities.

4. A formulation as claimed in any of claims 1 to 3, wherein neuropathic pain is painful diabetic neuropathy, postherpetic neuralgia, trigeminal neuralgia, phantom limb pain, causalgia, cancerous pain, or postoperative or traumatic chronic pain.

5. A formulation as claimed in any of claims 1 to 4, which can be used in combination with one or more of drugs selected from a group consisting of a psychotropic vitamins, a non-steroidal anti-inflammatory drug, an aldose reductase inhibitor, a lidocaine-like anti-arrhythmic drug, an antidepressant and an anticonvulsant.

6. A method of the prevention or treatment of neuropathic pain, which comprises repeated administration of effective doses of a β₂-adrenoceptor stimulant for 2 weeks or longer.

7. A method of the prevention or treatment of neuropathic pain; which comprises administration of effective doses of the combination of an α₂-adrenoceptor stimulant and a β₂-adrenoceptor stimulant.

8. A use of a β₂-adrenoceptor stimulant for the manufacture of a formulation for the prevention or treatment of neuropathic pain.

9. A use of an α₂-adrenoceptor stimulant and a β₂-adrenoceptor stimulant for the manufacture of a formulation the prevention or treatment of neuropathic pain.
